# EUROPEAN PATENT SPECIFICATION

(11) **EP 4 303 310 B1**
(45) Date of publication and mention of the grant of the patent: **24.12.2025**
(21) Application number: 22763119.9
(22) Date of filing: 25.02.2022
(51) Int. Cl.: C12P 21/02, C07K 14/195, C12N 9/16, C12N 15/31

(54) **METHOD FOR PRODUCING CAS3 PROTEIN**
VERFAHREN ZUR HERSTELLUNG VON CAS3-PROTEIN
PROCÉDÉ DE PRODUCTION DE PROTÉINE CAS3

(30) Priority: 01.03.2021 JP 2021031907
(43) Date of publication of application: 10.01.2024
(73) Proprietor: C4U Corporation, Suita-shi, Osaka, 565-0871 (JP); The University of Tokyo, Bunkyo-ku, Tokyo 113-8654 (JP); RIKEN, Wako-shi, Saitama 351-0198 (JP)
(72) Inventor: MASHIMO Tomoji, Tokyo 113-8654 (JP); YOSHIMI Kazuto, Tokyo 113-8654 (JP); TAKESHITA Kohei, Suita-shi, Osaka 565-0871 (JP); YAMAMOTO Masaki, Wako-shi, Saitama 351-0198 (JP); SHIBUMURA Satomi, Suita-shi, Osaka 565-0871 (JP)
(74) Representative: J A Kemp LLP
(86) International application number: PCT/JP2022/007821
(87) International publication number: WO 2022/186063

(56) References cited:
- WO-A1-2021/149829
- JP-A- 2004 283 111
- JP-A- 2010 053 132
- JP-A- H06 277 088
- JP-B1- 6 940 086
- SINKUNAS TOMAS ET AL: "Cas3 is a single-stranded DNA nuclease and ATP-dependent helicase in the CRISPR/Cas immune system", EMBO (EUROPEAN MOLECULAR BIOLOGY ORGANIZATION) JOURNAL,, vol. 30, no. 7, 1 April 2011 (2011-04-01), pages 1335 - 1342, XP002765626, DOI: 10.1038/EMBOJ.2011.41
- CONTRERAS-G�MEZ A ET AL: "Protein production using the baculovirus-insect cell expression system", BIOTECHNOLOGY PROGRESS, AMERICAN CHEMICAL SOCIETY, HOBOKEN, USA, vol. 30, no. 1, 1 December 2013 (2013-12-01), pages 1 - 18, XP072298015, ISSN: 8756-7938, DOI: 10.1002/BTPR.1842
- YOSHIMI KAZUTO ET AL: "Dynamic mechanisms of CRISPR interference by Escherichia coli CRISPR-Cas3", vol. 13, no. 1, 30 August 2022 (2022-08-30), UK, XP093180302, ISSN: 2041-1723, Retrieved from the Internet <URL:https://www.nature.com/articles/s41467-022-32618-0> DOI: 10.1038/s41467-022-32618-0
- SINKUNAS TOMAS ET AL: "Cas3 is a single-stranded DNA nuclease and ATP-dependent helicase in the CRISPR/Cas immune system", EMBO (EUROPEAN MOLECULAR BIOLOGY ORGANIZATION) JOURNAL, vol. 30, no. 7, 1 April 2011 (2011-04-01), pages 1335 - 1342, XP002765626, DOI: 10.1038/emboj.2011.41
- KAWAGUCHI YUKO, OKAMOTO, KEN: "3. Gene Delivery and Expression Series. Overexpression of Recombinant Protein in Large-scale Culture Using a Baculovirus-insect Cell Expression System: Based on the Study of Xanthine Oxidoreductase (3)), non-official translation", NIHON IKA DAIGAKU IGAKKAI ZASSHI, vol. 8, no. 1, 1 January 2012 (2012-01-01), pages 26 - 30, XP055965066, ISSN: 1349-8975
- HIDEKI YAMAJI: "Production of Useful Substances Using Insect Cells", SEIBUTSU KOGAKU KAISHI = JOURNAL OF THE SOCIETY FOR BIOSCIENCE AND BIOENGINEERING, JAPAN, NIPPON SEIBUTSU KOGAKKAI, SUITA,, JP, vol. 94, no. 2, 1 January 2016 (2016-01-01), JP , pages 76 - 80, XP055965072, ISSN: 0919-3758
- JAMIESON A. L. HOWARD, STEPHANE DELMAS, IVANA IVAN&CCARON;I&CACUTE;&#X2011;BA&CACUTE;E, EDWARD L. BOLT: "Helicase dissociation and annealing of RNA-DNA hybrids by Escherichia coli Cas3 protein", BIOCHEMICAL JOURNAL, PUBLISHED BY PORTLAND PRESS ON BEHALF OF THE BIOCHEMICAL SOCIETY., vol. 9, no. 1, 1 October 2011 (2011-10-01), pages 4163 - 95, XP055053995, ISSN: 02646021, DOI: 10.1042/BJ20110901
- IVANČIĆ-BAĆE IVANA, RADOVČIĆ MARIN, BOČKOR LUKA, HOWARD JAMIESON L., BOLT EDWARD L.: "Cas3 stimulates runaway replication of a ColE1 plasmid in Escherichia coli and antagonises RNaseHI", RNA BIOLOGY, vol. 10, no. 5, 1 May 2013 (2013-05-01), pages 770 - 778, XP055965083, ISSN: 1547-6286, DOI: 10.4161/rna.23876
- MAJSEC KRISTINA, BOLT EDWARD L., IVANČIĆ-BAĆE IVANA: "Cas3 is a limiting factor for CRISPR-Cas immunity in Escherichia coli cells lacking H-NS", BMC MICROBIOLOGY, vol. 16, no. 1, 1 December 2016 (2016-12-01), XP055965085, DOI: 10.1186/s12866-016-0643-5

## Description

### [Technical Field]

The present invention relates to a method for producing Cas3 proteins, and more specifically relates to a method for producing recombinant Cas3 proteins with maintained activity, in high purity and high yield.

### [Background Art]

Genome editing technology is a technique that specifically cleaves the genomic DNA sequence in the cells of animals and plants, and freely rewrites it to any sequence by utilizing the inherent repair mechanism. Its use is spreading worldwide, not only in bioscience research, but also in crop and livestock breed improvement, regenerative medicine, gene therapy, and the like.

The CRISPR-Cas system, possessed by bacteria and archaea, is divided into Class 1, which cleaves the target sequence by a complex of multiple proteins, and Class 2, which cleaves by a single protein. CRISPR-Cas9, CRISPR-Cas12 (Cpf1), CRISPR-Cas13, and the like, which have been developed as genome editing tools so far, are all classified as Class 2, but recently, it has been found that CRISPR-Cas3, which is a Type I CRISPR belonging to Class 1, can be used as a genome editing tool in eukaryotic cells (PTL 1). In particular, it has been found that Type I-E CRISPR-Cas3 derived from E. coli K strain can recognize and bind to a target of 27 bases in addition to the 3-base PAM sequence, and can introduce wide-range deletion mutations of several hundred to several kb upstream of the target sequence in human cultured cells with high efficiency. Furthermore, it is considered to have high safety because the target recognition sequence in guide RNA is long compared to CRISPR-Cas9, and non-specific cleavage is less likely to occur.

CRISPR-Cas3 is generally used by introducing it in cells as an expression plasmid and expressing it. However, depending on the type of cell, its introduction and expression can be difficult, and in some cases, sufficient genome editing efficiency may not be achieved. For this reason, it is desirable to introduce CRISPR-Cas3 into cells in the form of guide RNA (crRNA) and proteins (Cas3 protein and Cascade protein).

However, among the group of proteins that constitute CRISPR-Cas3, particularly regarding Cas3 proteins, it has traditionally been difficult to prepare a practically viable active form in sufficient quality and quantity. For example, reports exist on the purification methods for existing Cas3 proteins, such as the Cas3 protein derived from the thermophile Thermobifida fusca (TfuCas3) and the Cas3 protein derived from the E. coli K strain (EcoCas3) (see NPLs 1 to 3), but TfuCas3 has the problem of not being suitable for use at the growth temperature of many cells due to its thermophilic origin. Meanwhile, EcoCas3, derived from E. coli, can demonstrate activity at a temperature range close to the growth temperature of many cells, including animal cells, and thus has properties suitable for genome editing of a wide range of organisms with potential industrial applications. However, even when trying to produce it with conventional methods, it has been difficult to purify it at high purity and high concentration with maintained activity (see Comparative Example 1 described later). WO2021/149829 [PTL 2] describes a method for detecting specific DNA in sample, which is based on the finding that mixing single-stranded probe DNA, the cleavage of which is detectable, in a reaction system containing a sample for detection of target DNA and a CRISPR-Cas3 system allows for the detection of the target DNA in the sample using, as an indicator, a signal generated by the cleavage of the single-stranded probe DNA.

Sinkunas et al. [NPL 4] reports that Cas3 is a single-stranded DNA nuclease and ATP-dependent helicase in the CRISPR/Cas immune system.

Contreras-Gómez et al. [NPL 5] reports protein production using the baculovirus-insect cell expression system.

### [Citation List]

### [Patent Literature]

[PTL 1] International Publication No. WO2018/225858
[PTL 2] International Publication No. WO2021/149829

### [Non Patent Literature]

[NPL 1] Huo Y. et al., Nat. Struct. Mol. Biol. 2014 Sep; 21(9): 771-777
[NPL 2] Mulepati S. & Bailey S., J. Biol. Chem. 2013 Aug 2; 288(31): 22184-22192
[NPL 3] Robert P. Hayes et al., Nature 2016 530: 499-503
[NPL 4] Sinkunas, Tomas, et al. The EMBO journal 30.7 (2011): 1335-1342.
[NPL 5] Contreras - Gómez, A., et al. Biotechnology progress 30.1 (2014): 1-18.

### [Summary of Invention]

The invention is defined in independent claim 1. Further aspects and preferred embodiments are defined in the dependent claims. Any aspects, embodiments and examples of the present disclosure which do not fall under the scope of the appended claims do not form part of the invention and are merely provided for illustrative purposes.

### [Technical Problem]

The present invention has been made in view of the issues with the aforementioned conventional techniques, and an object thereof is to provide a method for producing active forms of Cas3 proteins in high purity and high yield, which can be used for genome editing in various organisms.

### [Solution to Problem]

The present inventors have made earnest studies to solve the aforementioned issues, and have found that Cas3 proteins derived from E. coli have low thermal stability, and even when expressed as recombinant proteins at the normal culture temperature of E. coli, they denature, leading to a reduction in activity.

Based on this unexpected finding, the present inventors selected insect cells, which can be cultured at relatively low temperatures, and introduced the Cas3 gene to culture them under various temperature conditions. As a result, they found that by culturing at 20 to 28°C, the Cas3 protein was efficiently expressed with its activity maintained.

Furthermore, the present inventors conducted an examination of the purification conditions for recombinant Cas3 proteins expressed in insect cells, and discovered that purification in a phosphate buffer made it possible to collect the active forms of the recombinant Cas3 proteins in high purity and high yield.

Furthermore, the present inventors discovered that the recombinant Cas3 proteins, thus collected, exhibited high activity even at the culture temperatures of animal cells, within a relatively short period of time required for genome editing. This finding led to the completion of the present invention.

In short, the present invention relates to a method for producing recombinant Cas3 proteins, which can be used for genome editing in various cells, with maintained activity in high purity and high concentration.

More specifically, the invention provides a method for producing a Cas3 protein, comprising:
(a) culturing a Cas3 gene-introduced insect cell at 20 to 28°C to express a Cas3 protein in the insect cell, wherein the insect cell is an Sf9 cell; and
(b) collecting the expressed Cas3 protein, wherein the collection of the expressed Cas3 protein includes purifying the Cas3 protein, wherein a buffer used for the purification is a phosphate buffer.

The Cas3 protein may be derived from E. coli.

The Cas3 protein may include a tag added thereto, and the purification of the Cas3 protein may include affinity purification with the tag.

The tag may include an HN tag.

The purification of the Cas3 protein may include purification by gel filtration chromatography.

### [Advantageous Effects of Invention]

According to the present invention, it is possible to produce active forms of recombinant Cas3 proteins in high purity and high yield. The method of the present invention can be used for the production of various recombinant Cas3 proteins, but is particularly useful for the application to Cas3 proteins with low thermal stability.

In addition, as described herein, it is possible to prepare the CRISPR-Cas3 system in a usable state before performing genome editing. Furthermore, even in cells where it is difficult to introduce and express Cas3 as a gene or where sufficient genome editing efficiency cannot be achieved when Cas3 is expressed as a recombinant protein, it is possible to perform genome editing efficiently. For this reason, it is possible to use the CRISPR-Cas3 system in a simple and universal way.

### [Brief Description of Drawings]

[Fig. 1] Fig. 1 is a graph showing purification of recombinant EcoCas3 protein expressed in E. coli by gel filtration chromatography.
[Fig. 2] Fig. 2 is an electrophoresis photograph showing recombinant EcoCas3 protein expression in insect cells cultured at each temperature.
[Fig. 3] Fig. 3 shows a graph (top) illustrating the results of purifying recombinant EcoCas3 protein expressed in insect cells by gel filtration chromatography using a phosphate buffer, as well as a photograph of SDS-PAGE (bottom). The lanes in SDS-PAGE are as follows: a. Supernatant, b. Flow-through, c. TEV digestion, d. Wash, e. Flow-through of backtrap, 2-20. SEC fractions, f. Concentrated fractions 13-16.
[Fig. 4] Fig. 4 is a graph (top) illustrating the results of purifying recombinant EcoCas3 protein expressed in insect cells by gel filtration chromatography using a Hepes buffer, as well as a photograph of SDS-PAGE (bottom).
[Fig. 5] Fig. 5 is a graph showing the inflection point temperature due to thermal denaturation of the recombinant EcoCas3 protein. The inflection point temperature was measured by TychoNT6.
[Fig. 6] Fig. 6 is a graph showing the stability of EcoCas3, Cas9, Cas12, and TfuCas3 at 37°C. The stability of these proteins was measured by the change in fluorescence intensity caused by Sypro_orange binding to the hydrophobic regions exposed to the solvent surface as the protein denatures.
[Fig. 7] Fig. 7 is a diagram showing a plasmid used for the production of the Multi-NLS EcoCascade.
[Fig. 8] Fig. 8 is a photograph of capillary electrophoresis showing the results of measuring genome editing activity in vitro using the purified Cas3 protein and a complex of Cascade protein and crRNA. The red arrow indicates the degradation of detected DNA. The left side presents the results using the EMX1 targeted Cascade, while the right side displays the results with the Tyr targeted Cascade.
[Fig. 9] Fig. 9 shows the results of measuring genome editing activity in human cultured cells using the purified Cas3 protein and a complex of Cascade protein and crRNA. The top part illustrates representative images obtained from FACS analysis, and the bottom part depicts a graph (n = 3) showing the analysis results of GFP knockout efficiency calculated from the number of GFP-negative cells.

### [Detailed Description]

The present invention provides a method for producing Cas3 proteins, comprising:
(a) culturing a Cas3 gene-introduced insect cell at 20 to 28°C to express a Cas3 protein in the insect cell, wherein the insect cell is an Sf9 cell; and
(b) collecting the expressed Cas3 protein, wherein the collection of the expressed Cas3 protein includes purifying the Cas3 protein, wherein a buffer used for the purification is a phosphate buffer.

As used herein, the term "Cas3 protein" refers to a protein that constitutes the CRISPR-Cas3 system and possesses nuclease activity and helicase activity. By cooperating with the cascade and crRNA that constitute the CRISPR-Cas3 system, the Cas3 protein is capable of cleaving the target DNA.

The Type I-E CRISPR-Cas3 system, which is common within Type I CRISPR-Cas3 systems, cleaves DNA by allowing crRNA to work in conjunction with Cas3 and cascades (Cse1 (Cas8), Cse2 (Cas11), Cas5, Cas6, and Cas7).

As constituent components, the Type I-A system includes the cascades Cas8a1, Csa5 (Cas11), Cas5, Cas6, and Cas7, the Type I-B system includes the cascades Cas8b1, Cas5, Cas6, and Cas7, the Type I-C system includes the cascades Cas8c, Cas5, and Cas7, the Type I-D system includes the cascades Cas10d, Csc1 (Cas5), Cas6, and Csc2 (Cas7), the Type I-F system includes the cascades Csy1 (Cas8f), Csy2 (Cas5), Cas6, and Csy3 (Cas7), and the Type I-G system includes the cascades Cst1 (Cas8a1), Cas5, Cas6, and Cst2 (Cas7).

The Cas3 protein described herein, regardless of its origin, is preferably a Cas3 protein derived from E. coli, from the viewpoint of its suitability for genome editing in a wide range of cells, including animal cells. The amino acid sequence of a typical E. coli-derived Cas3 protein is set forth in SEQ ID NO: 2, and the nucleotide sequence of the DNA encoding this protein is set forth in SEQ ID NO: 1. The Cas3 protein described herein includes mutants that have occurred in nature or have been artificially modified.

The Cas3 protein described herein may be a protein composed of an amino acid sequence that has high identity with the amino acid sequence of the E. coli-derived Cas3 protein set forth in SEQ ID NO: 1. High identity, for example, is 80% or more, preferably 85% or more, more preferably 90% or more (for example, 91% or more, 92% or more, 93% or more, 94% or more), and further preferably 95% or more (for example, 96% or more, 97% or more, 98% or more, 99% or more) sequence identity. Sequence identity can be determined using tools such as BLAST (Basic Local Alignment Search Tool at the National Center for Biological Information) (with default, or initially set parameters, for example). Additionally, the Cas3 protein described herein could be a protein composed of an amino acid sequence in which one or more amino acids in the amino acid sequence of the E. coli-derived Cas3 protein set forth in SEQ ID NO: 1 are substituted, deleted, added, and/or inserted. Here, "more" typically means within 50 amino acids, preferably within 30 amino acids, more preferably within 20 amino acids, and particularly preferably within 10 amino acids (for example, within 5 amino acids, within 3 amino acids, within 2 amino acids, 1 amino acid).

Cas3 proteins may, if necessary, be further supplemented with functional molecules. Examples of such functional molecules include, but are not limited to, nuclear localization signals to facilitate migration into the nucleus of eukaryotic cells, tags to simplify purification, and reporter proteins to ease detection. These functional molecules can, for example, be attached to the N-terminus and/or C-terminus of the Cas3 protein.

Examples of nuclear localization signals include PKKKRKV (set forth in SEQ ID NO: 3) and KRTADGSEFESPKKKRKV (set forth in SEQ ID NO: 4). Examples of tags include HN tag, His tag, FLAG tag, and glutathione S-transferase (GST) tag. Furthermore, examples of reporter proteins include fluorescent proteins such as green fluorescent protein (GFP), and chemiluminescent proteins such as luciferase.

In the production method described herein, insect cells, into which the Cas3 gene has been introduced, are cultured at 20 to 28°C to express the Cas3 protein within these insect cells (step (a)).

A method for expressing recombinant Cas3 protein in insect cells may involve the use of a known baculovirus expression system. In one example of using a baculovirus expression system, the Cas3 gene is first cloned into a Bac-to-Bac vector such as pFastBac1, and then introduced into E. coli having baculovirus DNA to prepare baculovirus DNA containing the Cas3 gene. Note that in addition to this method of preparing recombinant baculovirus DNA in E. coli, it is also possible to use a method of preparation in insect cells. When using insect cells, it suffices that a vector containing the Cas3 gene and baculovirus DNA are introduced into the insect cells, and homologous recombination between the two is allowed to occur. Next, the prepared recombinant baculovirus DNA is transfected into insect cells to prepare recombinant baculovirus containing the Cas3 gene. Subsequently, the prepared recombinant baculovirus is passage infected in insect cells to obtain a high-titer baculovirus, and this virus is infected into insect cells to express the recombinant Cas3 protein.

The cultivation of insect cells for the expression of recombinant Cas3 proteins is preferably conducted at 20 to 28°C. Below 20°C, there tends to be a decrease in the efficiency of expressing recombinant Cas3 proteins, while above 28°C, the recombinant Cas3 proteins expressed during the cultivation process tend to denature. From the viewpoint of further inhibiting denaturation, 20 to 24°C is more preferable, 20 to 22°C is further preferable, and 20°C is particularly preferable. The cultivation time is not particularly limited as long as it is sufficient for the expression of the recombinant Cas3 proteins, but it is usually 24 hours or longer, and preferably 60 and 72 hours.

In the production method described herein, the next step involves collecting the expressed Cas3 protein (step (b)).

In the collection of the expressed Cas3 proteins, various protein separation and purification methods can be used. In the separation of recombinant Cas3 proteins from the cells, cell disruption treatment and centrifugation can be used. For example, cells can be disrupted by ultrasonication, followed by centrifugation at 100,000 g, and by collecting the supernatant, a soluble fraction containing recombinant Cas3 proteins can be obtained.

When a tag is added to the Cas3 protein, affinity purification targeting this tag can be used in the purification of recombinant Cas3 proteins. For example, if the tag is an HN tag or His tag, a nickel column can be used; if it is a FLAG tag, beads bound with antibodies against the FLAG tag can be used; if it is a glutathione S-transferase (GST) tag, Glutathione Sepharose can be used, to thereby conduct affinity purification. From the viewpoint of electrically neutralizing the Cas3 protein and inhibiting aggregation, the HN tag is preferable as a tag to be added to the Cas3 protein.

Furthermore, it is preferable that the purification of the recombinant Cas3 protein includes purification by gel filtration chromatography. Since the Cas3 protein is a globular protein of approximately 100 kDa molecular weight, it is preferable to select a column with a fractionation range suitable for globular proteins of such molecular weight. As such a column, it is possible to use commercially available products such as Superdex 200 Increase (from Cytiva).

From the viewpoint of preventing aggregation due to the denaturation of Cas3 proteins, a phosphate buffer is preferable for use in purification.

The recombinant Cas3 protein thus prepared has excellent activity, and can demonstrate its activity without denaturation even under temperature conditions of 37°C, as long as it is within relatively a short period of time, the few hours necessary for genome editing. In fact, superior DNA cleavage activity and high genome editing efficiency were observed at 37°C. Therefore, the recombinant Cas3 protein obtained by the method described herein can efficiently perform genome editing in a wide range of cells when combined with Cascade proteins and crRNA.

### [Examples]

### [Comparative Example 1] Preparation of EcoCas3 Protein Using E. Coli

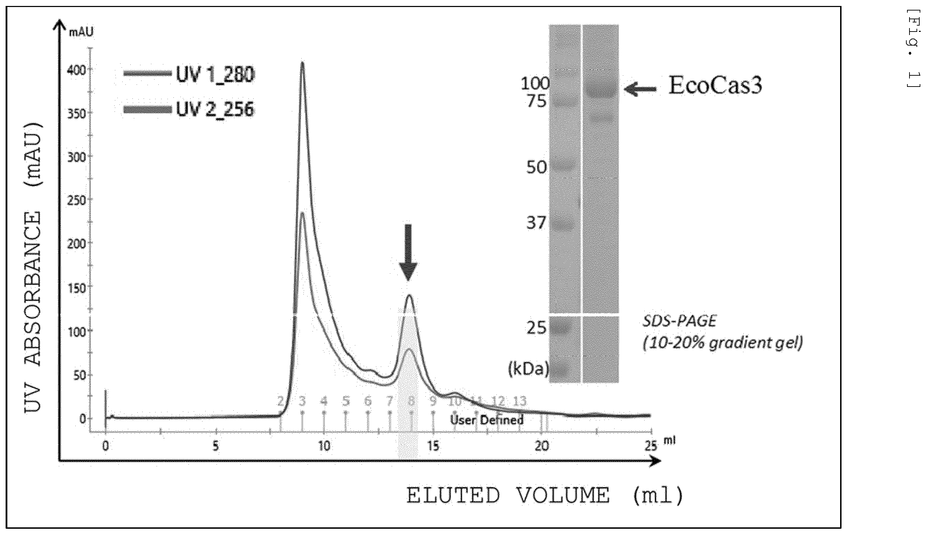

The conventional method for producing EcoCas3 (Mulepati S. & Bailey S., J Biol Chem. 2013 Aug 2; 288(31): 22184-22192) has a number of problems such as: (i) E. coli, into which the plasmid encoding EcoCas3 has been introduced, must be cultured at the low temperature of 20°C, (ii) in order to maintain the solubility of EcoCas3, it is necessary to fuse maltose-binding protein (MBP) or small ubiquitin-like modifier (SUMO) to EcoCas3, (iii) it is also necessary to coexpress the chaperone molecule, HtpG protein, (iv) the yield is at most 1 mg per liter of culture, and (v) it is difficult to produce high-purity EcoCas3 as protein bands other than EcoCas3 can be confirmed as the electrophoresis pattern. In fact, when EcoCas3 proteins were prepared using E. coli according to the above literature, it was low in purity and yield (Fig. 1), and its activity was also extremely low.

### [Example 1] Preparation of EcoCas3 Protein Using Insect Cell Sf9

When a protein derived from a prokaryotic organism is expressed as a recombinant protein in a higher eukaryotic organism, post-translational modifications that do not occur in prokaryotic organisms might take place. Therefore, the production of EcoCas3 proteins in eukaryotic organisms has not been conducted previously, but in light of the challenges described in Comparative Example 1, the present inventors dared to attempt the preparation of EcoCas3 proteins using insect cells Sf9.

### (1) Construction of Plasmid for Expression of Recombinant EcoCas3 Protein

A gene was synthesized having an 8HN tag (a tag having a His tag and an HN tag fused with a GS linker in between/set forth in SEQ ID NO: 5) and an NLS (set forth in SEQ ID NO: 3) fused at the N-terminus of EcoCas3, and further having an NLS (set forth in SEQ ID NO: 3) fused at the C-terminus as well (SEQ ID NOs: 6 and 7). The use of the HN tag (a repeat sequence of histidine and asparagine) was to neutralize the strong positive charge bias of the His tag, which had the potential to aggregate the target protein. Additionally, for expression confirmation, a gene was also created having EGFP as a reporter fused at the 3'-terminus of the 8HN tag (SEQ ID NOs: 8 and 9).

The aforementioned fusion gene was cloned into the pFastbac-1 plasmid (manufactured by ThermoFishers). The resulting EcoCas3/pFastbac-1 plasmid was transformed into DH10bac, and after incorporating it into the baculovirus genome in DH10bac by homologous recombination, the baculovirus genome containing the EcoCas3 gene was extracted.

### (2) Expression of Recombinant EcoCas3 Protein

A baculovirus genome containing the EcoCas3 gene or the EGFP-fused EcoCas3 gene was transfected into Sf9 cells, and a baculovirus containing the EcoCas3 gene was produced within the Sf9 cells. This baculovirus was passage infected in Sf9 cells, resulting in the acquisition of a high-titer virus for EcoCas3 expression. This high-titer virus was infected into Sf9 cells, leading to the expression of EcoCas3 as a recombinant protein.

In the examination of the expression of recombinant EcoCas3 proteins, a baculovirus containing the EGFP-fused EcoCas3 gene was used, and infection of the baculovirus into Sf9 cells was conducted at 28°C for 24 hours, after which the Sf9 cells were cultured at various culture temperatures (from 12°C to 28°C) for 60 hours to express the recombinant EcoCas3 protein. The cells were disrupted by ultrasonication, and the supernatant (soluble fraction) collected by centrifugation at 100,000 g was subjected to electrophoresis and fluorescence detection.

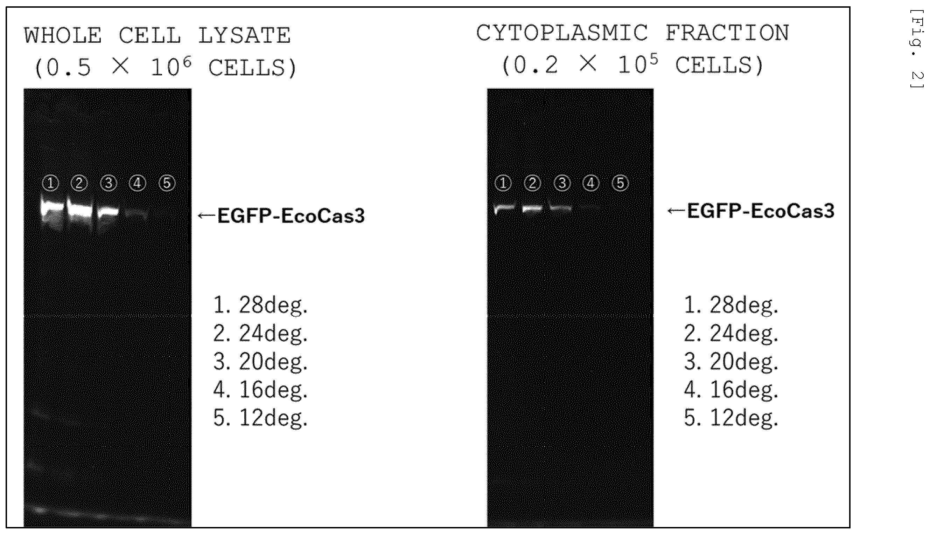

As a result, the expression of recombinant EcoCas3 protein in the soluble fraction was confirmed under temperature conditions of 16°C or higher, and the expression efficiency in the soluble fraction was particularly high under temperature conditions of 20°C or higher (Fig. 2). In the following experiments, recombinant EcoCas3 protein, which was expressed in Sf9 cells under temperature conditions of 20°C, was used.

### (3) Preparation of Recombinant EcoCas3 Protein

The recombinant EcoCas3 protein, supplemented with a His tag-like 8HN tag, was affinity-purified using a nickel column, followed by final purification through gel filtration chromatography.

Insect cells expressing recombinant EcoCas3 protein were disrupted by ultrasonication. The supernatant (soluble fraction) was collected by centrifugation at 100,000 g and mixed with nickel agarose resin (Qiagen) to bind the recombinant EcoCas3 protein to the resin. This was then washed with a wash buffer (20 mM HEPES or 20 mM KH₂PO₄, 350 mM NaCl, 40 mM imidazole, 0.5 mM DTT, pH 7.0). Following this, an elution buffer (20 mM Hepes or 20 mM KH₂PO₄, 350 mM NaCl, 200 mM imidazole, 0.5 mM DTT, pH 7.0) was used to elute the recombinant EcoCas3 protein from the resin.

By subjecting the elution fraction to TEV digestion treatment, the 8HN tag added to the N-terminus of the recombinant EcoCas3 protein was cleaved off. Finally, the final purification of the recombinant EcoCas3 protein was conducted using gel filtration chromatography. The purification was conducted using a Superdex 200 increase column (Cytiva), and the mobile phase buffer used was "20 mM HEPES or 20 mM KH₂PO₄, 200 mM NaCl, 1.0 mM DTT, pH 7.0".

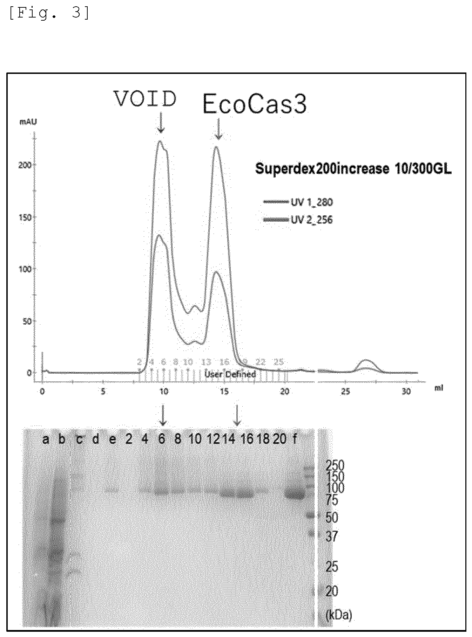

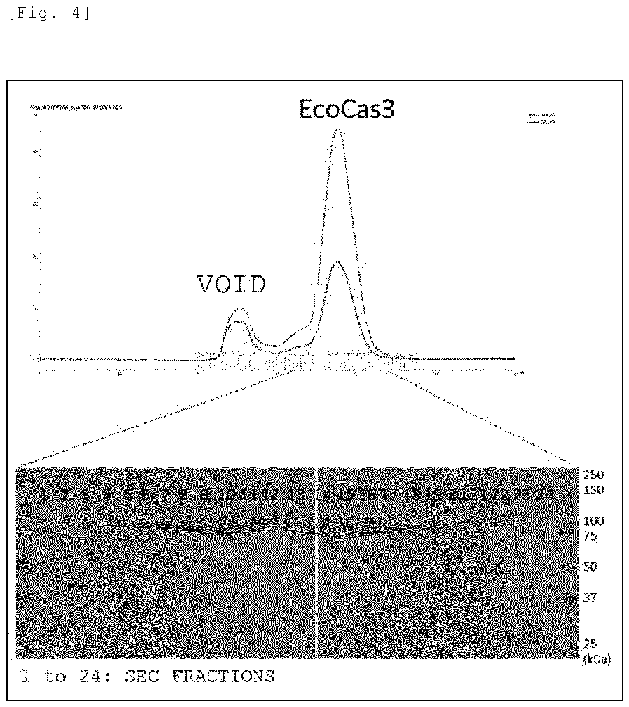

As a result, when purification was conducted using a HEPES buffer, a high number of void fractions containing EcoCas3, believed to have aggregated in gel filtration chromatography, were observed (Fig. 3). Meanwhile, when purification was conducted using a phosphate buffer, the inflection point temperature Ti improved by approximately 2°C, and ultimately, at most 2 mg of purified EcoCas3 protein per 1L culture was successfully obtained (Fig. 4). It is believed that the use of the phosphate buffer suppressed the separation of EcoCas3 to the void fractions. The purity was verified by SDS-PAGE, and no protein bands other than the target EcoCas3 protein were detected. This revealed that it was significantly more highly purified than the conventional EcoCas3 protein purified using the E.coli expression system.

### [Example 2] Measurement of Thermal Stability of Recombinant EcoCas3 Protein

The thermal stability of the recombinant EcoCas3 protein was evaluated by measuring the inflection point temperature Ti of the thermal denaturation profile using TychoNT6 (manufactured by NanoTemper). The peak shift of the intrinsic fluorescence derived from intramolecular tryptophan residues, accompanying the thermal unfolding of protein molecules, was detected at two wavelengths, 330 nm and 350 nm, and the ratio of these fluorescence intensities was plotted against temperature to determine the inflection point temperature Ti of the thermal denaturation profile.

The thermal stability of the recombinant EcoCas3 protein was also measured using SYPRO Orange fluorescent reagent. SYPRO Orange exhibits fluorescence by binding to the denatured hydrophobic regions of the protein. The binding of SYPRO Orange and the increase in fluorescence intensity, which depend on the exposure of the protein's hydrophobic regions to the solvent due to structural changes with thermal denaturation, were detected using a real-time PCR device. Fluorescence detection was conducted at an excitation wavelength of 473 nm and a fluorescence wavelength of 520 nm.

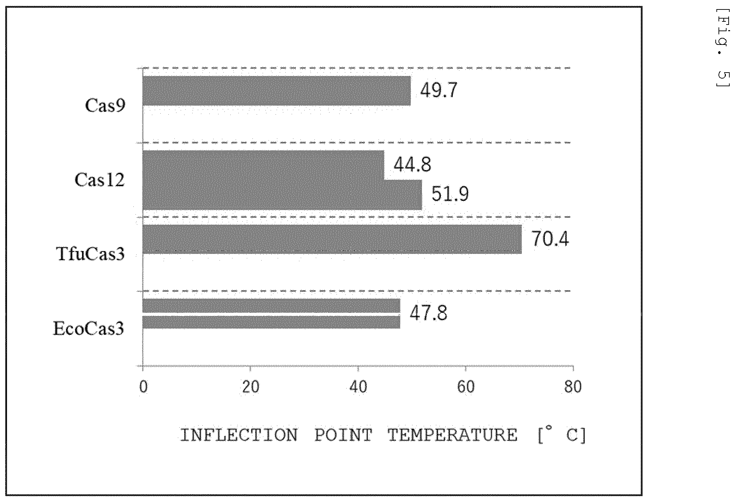

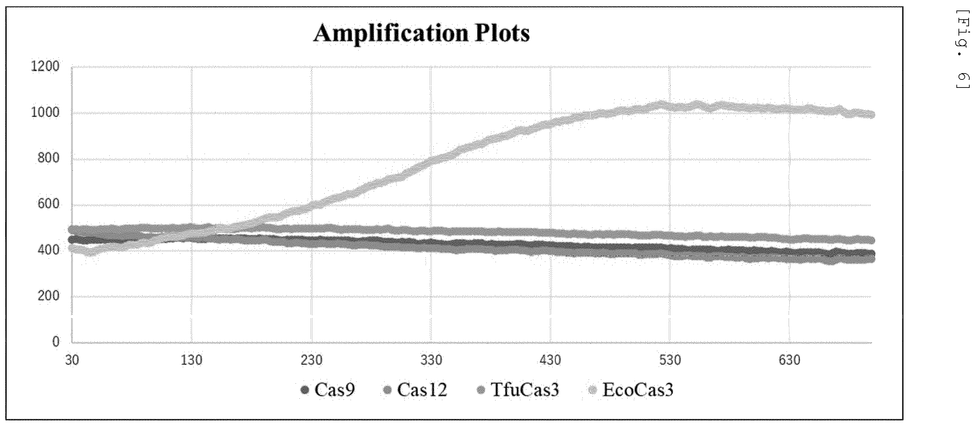

It was found from the results above that in measurements using TychoNT6, the inflection point temperature of the recombinant EcoCas3 protein was roughly equivalent to that of Cas9 (Fig. 5), but in stability measurements at a constant temperature of 37°C using the SYPRO Orange fluorescent reagent, it was found that the recombinant EcoCas3 protein completely denatured after about 8 hours (Fig. 6). It is considered from these results that the recombinant EcoCas3 protein is in a state of high entropy, indicating the molecular disorder, and has significant fluctuations. Consequently, the change in Gibbs free energy, a thermodynamic parameter, is small, and it is considered to be less stable compared to Cas9, Cas12, and TfuCas3. These results on the stability of the recombinant EcoCas3 protein well explain the result (from Example 1(2)) that this protein can be highly efficiently expressed at a temperature lower than the usual insect cell culture temperature (28°C) in Sf9 cells. Meanwhile, it was found that the purified recombinant Cas3 protein retained high activity for several hours at 37°C. This fact well explains that it can be used without any particular problem in the in vitro and various cell-based cleavage processing of target DNA (results of Example 3 and 4 described later).

### [Example 3] Preparation of EcoCascade Complex

To conduct genome editing by introducing recombinant EcoCascade into cells simultaneously with recombinant EcoCas3 proteins, it is crucial to efficiently transport them intracellularly into the nucleus. However, the EcoCascade, with a molecular weight of as much as 0.4 MDa approximately, raises concerns about its nuclear import rate. Therefore, the present inventors divided the operon of the five genes constituting the EcoCascade into two parts, and by attaching NLS to the 3'-terminus of each, attempted to introduce more NLS into the Cascade for a highly efficient transition into the cell nucleus.

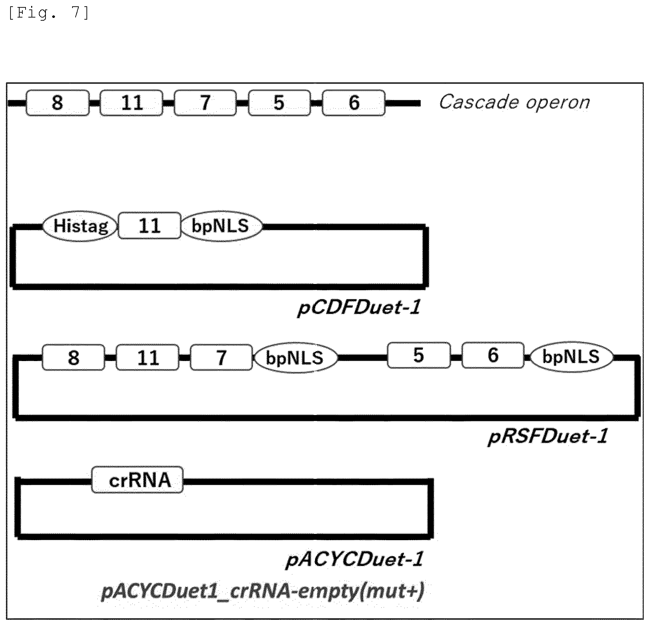

The EcoCascade is a supramolecular complex composed of Cas8-Cas11-Cas7-Cas5-Cas6. It is composed of one Cas8 molecule, two Cas11 molecules, five Cas7 molecules, two Cas5 molecules, and one Cas6 molecule. The present inventors constructed three plasmids: one having a His-tag-fused Cas11-NLS incorporated in pCDFuet-1 plasmid, another having the "Cas8-Cas11-Cas7 operon" with an NLS added at the 3'-terminus and the "Cas5-Cas6 operon" with an NLS added at the 3'-terminus incorporated in pRSFDuet-1 plasmid, and the last having crRNA incorporated in pACYCDuet-1 (Fig. 7, set forth in SEQ ID NOs: 10 to 24).

These three plasmids were incorporated into E. coli JM109 (DE3) (a lysogenic E. coli that carries bacteriophage λDE3 incorporated with the T7 RNA polymerase gene controlled by the lacUV5 promoter), and the recombinant protein or crRNA was expressed using IPTG. Through the His tag introduced into Cas11, affinity purification of the Multi-NLS-EcoCascade was conducted using a nickel column, followed by purification through gel filtration chromatography. As a result, approximately 1 mg of Multi-NLS-EcoCascade was successfully produced from 2L of culture.

Note that the target sequences of crRNA were set as sequences within the human EMX1 gene, the mouse Tyr gene, and the Aequorea victoria GFP gene.

### [Example 4] In Vitro Measurement of DNA Cleavage

A double-stranded DNA was used to examine the cleavage activity of target DNA in vitro, with purified EcoCas3 and EcoCascade proteins. In a reaction buffer (5 mM HEPES-K pH 7.5, 60 mM KCl, 10 mM MgCl₂, 10 µM CoCl₂, 2.5 mM ATP), Cas3 protein (20 nM), a complex of crRNA and Cascade (20 nM), and double-stranded DNA (60ng/µL) containing the target sequence were mixed. This reaction solution was incubated at 37°C for one hour, and capillary electrophoresis was conducted using MultiNa (Shimadzu Corporation). The target sequences were the EMX1 gene region and the Tyr gene region. Note that for EMX1, a sequence was also prepared and examined, changing the PAM sequence of the double-stranded DNA from "AAG", which can be recognized by type I-E CRISPR derived from E. coli, to "CCA", which cannot be recognized.

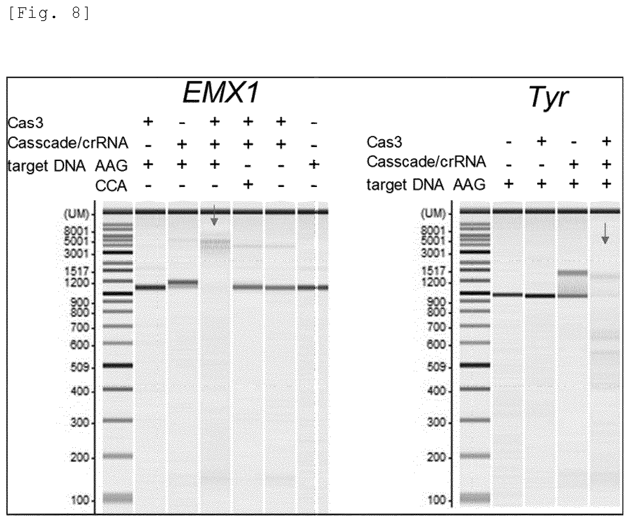

As a result, when a double-stranded donor DNA containing the target sequence of AAG recognizable by the PAM sequence was mixed, degradation of the donor DNA was observed (Fig. 8). Meanwhile, when donor DNA, changed to CCA unrecognizable by the PAM sequence, was mixed in, no DNA degradation was observed. Neither the Cas3 protein alone nor the Cascade complex alone exhibited DNA cleavage activity. Meanwhile, in the case of the Cascade complex alone, the band was detected higher than usual. This is believed to occur because the Cascade complex recognizes and binds to the DNA, thus under non-denaturing conditions, it becomes larger than usual, causing a gel shift.

### [Example 5] Activity Measurement in Cultured Human HEK293T Cells

Reporter HEK293T cells with mCherry-P2A-EGFP were used to examine the mutation introduction efficiency of purified EcoCas3 and EcoCascade proteins in human cells. Cas3 protein (30 µM or 45 µM) and a complex of GFP-targeting crRNA and Cascade (30 µM or 45 µM) were introduced into the reporter cells by electroporation using Neon Transfection System (Thermo Fisher Scientific). After cultivating the cells at 37°C in 5% CO₂ for five days, all cells were collected and the number of GFP-negative cells was counted using SH800 (SONY) to calculate the mutation introduction efficiency.

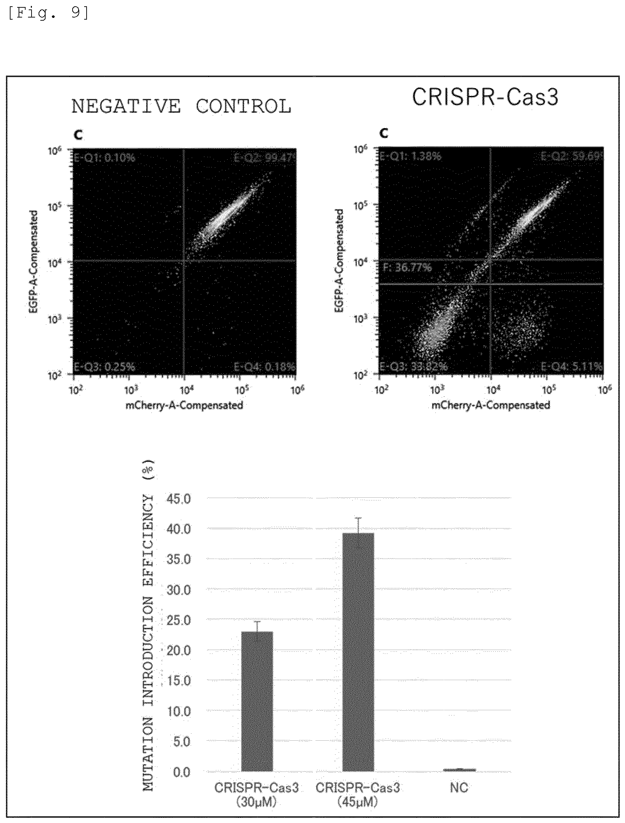

As a result of counting, when introduced at 30 µM, approximately 20% of the cells were GFP-negative, and when introduced at 45 µM, approximately 40% of the cells were GFP-negative (Fig. 9). Specifically, this demonstrated that genome editing using the CRISPR-Cas3 protein could be conducted with high efficiency, up to approximately 40%.

### [Industrial Applicability]

Cas3 proteins produced by the method described herein can be used for genome editing in various cells, and can therefore be used not only in basic research but also in various fields of genome editing technology applications such as medicine, agriculture, and industry.

## Claims

1. A method for producing a Cas3 protein, comprising:
(a) culturing a Cas3 gene-introduced insect cell at 20 to 28°C to express a Cas3 protein in the insect cell, wherein the insect cell is an Sf9 cell; and
(b) collecting the expressed Cas3 protein, wherein the collection of the expressed Cas3 protein includes purifying the Cas3 protein, wherein a buffer used for the purification is a phosphate buffer.

2. The method according to claim 1, wherein the Cas3 protein is derived from E. coli.

3. The method according to claim 1 or 2, wherein the Cas3 protein includes a tag added thereto, and the purification of the Cas3 protein includes affinity purification with the tag.

4. The method according to claim 3, wherein the tag includes an HN tag.

5. The method according to any one of claims 1 to 4, wherein the purification of the Cas3 protein includes purification by gel filtration chromatography.

## Patentansprüche

1. Verfahren zur Herstellung eines Cas3-Proteins, das umfasst:
(a) Züchten einer mit dem Cas3-Gen eingeführten Insektenzelle bei 20 bis 28°C, um ein Cas3-Protein in der Insektenzelle zu exprimieren, wobei die Insektenzelle eine Sf9-Zelle ist; und
(b) Sammeln des exprimierten Cas3-Proteins, wobei das Sammeln des exprimierten Cas3-Proteins das Reinigen des Cas3-Proteins umfasst, wobei ein für die Reinigung verwendeter Puffer ein Phosphatpuffer ist.

2. Verfahren nach Anspruch 1, wobei das Cas3-Protein aus E. coli gewonnen wird.

3. Verfahren nach Anspruch 1 oder 2, wobei das Cas3-Protein einen ihm hinzugefügten Tag enthält und die Reinigung des Cas3-Proteins eine Affinitätsreinigung mit dem Tag umfasst.

4. Verfahren nach Anspruch 3, wobei der Tag einen HN-Tag enthält.

5. Verfahren nach einem der Ansprüche 1 bis 4, wobei die Reinigung des Cas3-Proteins die Reinigung durch Gelfiltrationschromatographie umfasst.

## Revendications

1. Procédé pour produire une protéine Cas3, comprenant :
(a) la mise en culture d'une cellule d'insecte, introduite par gène Cas3, à 20 à 28°C pour exprimer une protéine Cas3 dans la cellule d'insecte, dans lequel la cellule d'insecte est une cellule Sf9 ; et
(b) la collecte de la protéine Cas3 exprimée, dans lequel la collecte de la protéine Cas3 exprimée inclut la purification de la protéine Cas3, dans lequel un tampon utilisé pour la purification est un tampon phosphate.

2. Procédé selon la revendication **1,** dans lequel la protéine Cas3 est dérivée de E. coli.

3. Procédé selon la revendication 1 ou 2, dans lequel la protéine Cas3 inclut une étiquette ajoutée à celle-ci, et la purification de la protéine Cas3 inclut la purification par affinité avec l'étiquette.

4. Procédé selon la revendication 3, dans lequel l'étiquette inclut une étiquette HN.

5. Procédé selon l'une quelconque des revendications 1 à **4,** dans lequel la purification de la protéine Cas3 inclut la purification par chromatographie gel-filtration.
